# EUROPEAN PATENT SPECIFICATION

(11) **EP 2 828 360 B1**
(45) Date of publication and mention of the grant of the patent: **04.10.2017**
(21) Application number: 13765104.8
(22) Date of filing: 12.03.2013
(51) Int. Cl.: C07C 2/64, C07C 15/107, C07C 2/06, C07C 5/327, C07C 7/163, C07C 7/167, C07C 11/02, C10G 9/00, C10G 29/20, C10G 45/32, C10G 50/00, C10G 57/00, C10G 57/02, C10G 69/00

(54) **HEAVY ALKYLBENZENE PRODUCTION THROUGH OLIGOMERIZATION**
SCHWERE ALKYLBENZOLHERSTELLUNG DURCH OLIGOMERISIERUNG
PRODUCTION D'ALKYLBENZÈNE LOURD PAR OLIGOMÉRISATION

(30) Priority: 23.03.2012 US 201213428559
(43) Date of publication of application: 28.01.2015
(73) Proprietor: UOP LLC, Des Plaines, Illinois 60017-5017 (US)
(72) Inventor: BOZZANO, Andrea G., Des Plaines, Illinois 60017-5017 (US); BRICKER, Jeffery C., Des Plaines, Illinois 60017-5017 (US); GLOVER, Bryan K., Des Plaines, Illinois 60017-5017 (US)
(74) Representative: Boult Wade Tennant
(86) International application number: PCT/US2013/030366
(87) International publication number: WO 2013/142137

(56) References cited:
- WO-A1-2009/027582
- WO-A1-2009/027582
- US-A- 4 990 718
- US-A- 4 990 718
- US-A1- 2009 292 150
- US-A1- 2010 305 373
- E. KRIVAN, ET AL.: "Investigation of the oligomerisation of light olefins on ion exchange resin catalyst", HUNGARIAN JOURNAL OF INDUSTRIAL CHEMISTRY, vol. 38, no. 1, 2010, pages 53-57, XP055034096, University of Veszprem, Research Institute of Chemical Engineering, HU ISSN: 0133-0276

## Description

### FIELD OF THE INVENTION

The present invention relates to the production of heavy olefins from a feedstock comprising C4 to C6 olefins, and the alkylation therewith of aromatic compounds.

### BACKGROUND OF THE INVENTION

High molecular weight olefins, also known as heavy olefins, are useful in many applications. Heavy olefins generally include olefins having carbon chains in the range from 18 to 30 carbon atoms. These olefins are useful in the production of surfactants for specialty applications. One area of growth for these specialty surfactants is enhanced oil recovery processes. As oil prices increase the economics of recovering oil from formerly marginal fields becomes favorable. Methods of recovering this oil include adding chemicals to improve the flow of the difficult to recover oil, and surfactants enhance the ability to get the oil to flow. The surfactants used in enhanced oil recovery processes can be recovered with the oil and can go through the normal processing of oil from an oil field.

US 4,990,718 describes aromatic alkylation with alpha-olefin dimer.

WO 2009/027582 describes a process for oligomerizing olefins.

The current process for generating heavy olefins is from processing heavy, or high molecular weight, paraffins. The heavy paraffins are separated from the heavy cuts from a crude oil feedstock, and the paraffins are then dehydrogenated to generate the heavy olefins. However, this process can be costly, and heavy paraffins can have other high value purposes, but the relatively low molecular weight C5 and C6 paraffins arc low value hydrocarbons and can be converted to higher value products.

It is desirable to use an inexpensive feedstock that can be converted to a more valuable product. Therefore, the conversion of lower value feeds containing C5 and C6 paraffins to higher value product streams comprising heavy olefins is desirable for forming a low cost material for use in enhanced oil recovery processes.

### SUMMARY OF THE INVENTION

The present invention provides a process for producing heavy olefins as defined in claim 1.

The present invention comprises integrating the production of heavy alkylbenzenes for the production of heavy surfactants. The heavy surfactants can be used in enhanced oil recovery processes. The process includes utilizing a feedstock having C4 to C6 olefins and oxygenated compounds. The feedstock is contacted with a liquid extractant to remove oxygenated compounds and generate a deoxygenated feedstock. The deoxygenated feedstock is passed to an oligomerization reactor, operated under chain growth conditions to generate a stream having heavy mono-olefins in the C 15 to C36 range. The olefins are passed to an alkylation reactor, along with an aromatic stream under alkylation conditions to generate an alkylaromatic compound process stream. The alkylaromatic compound process stream is passed to a separation unit to separate the aromatic compound from the alkylaromatic compound and to generate an alkylaromatic product stream and an aromatic stream for recycle.

Other objects, advantages and applications of the present invention will become apparent to those skilled in the art from the following detailed description and drawings.

### BRIEF DESCRIPTION OF THE DRAWING

Figure 1 shows the process of generating heavy olefins for aromatic alkylation; and
Figure 2 shows the process of generating heavy alkylaromatics from light olefins generated from a stream having olefins and oxygenates.

### DETAILED DESCRIPTION OF THE INVENTION

Surfactants have been used in chemical flooding systems for enhanced oil recovery processes. For enhanced oil recovery, higher molecular weight surfactants, or longer chained molecules are desirable. However, the production of surfactants is an expensive process. With increasing oil prices, the production has become more favorable, but producing surfactants through cheaper processes can improve the use of surfactants in enhanced oil recovery even at lower oil prices. With a shift in markets, and product specifications for gasoline and other products, there is an increase in lower value products such as pentane and hexane, as these hydrocarbon components are removed from commercial products. The process for increasing the value of low value hydrocarbons into higher value products is important and provides a means for producing high molecular weight alkylbenzenes from lower value hydrocarbons. The present invention allows for the production of large alkylbenzenes from renewable sources, and can utilize feedstocks that have an oxygenate component.

The present invention utilizes the use of a cheap feedstock comprising C4 to C6 olefins to produce heavy alkylbenzenes, and in particular heavy linear alkylbenzenes. For enhanced oil recovery, linearity is not required as biodegradability is not an issue. Therefore, larger alkyl groups can be used with significant branching. The process of the present invention allows for flexibility in creating heavy alkylbenzenes for use in surfactant manufacture. This allows for tailoring surfactant design for different oil fields, due to the different makeup of petroleum in different oil fields. The process includes generating olefins having between 15 and 36 carbon atoms, with a preferred range in the C18 to C28 range for the olefins.

In one embodiment, the source of the feedstock is from a light cracked naphtha. The light cracked naphtha can be processed to remove light olefins, i.e. ethylene and propylene, before passing the light cracked naphtha to the present process.

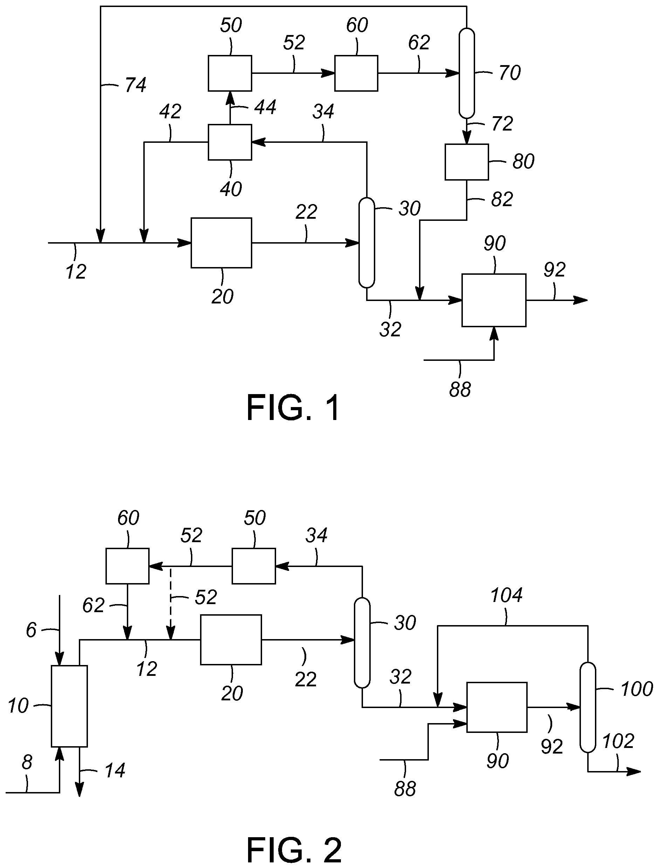

The process of the present invention is shown in Figure 1. The process includes passing a hydrocarbon feedstock 12 to an oligomerization reactor 20, to generate a first stream 22 comprising heavy olefins in the C15 to C36 range. The feedstock 12 comprises olefins having from 4 to 6 carbon atoms. The first stream 22 is passed to a first separation unit 30 to generate a product stream 32 comprising heavy olefins in the C15 to C36 range, and a recycle stream 34 comprising lighter hydrocarbon compounds, including lighter olefins.

The recycle stream 34 can be passed to the oligomerization reactor 20, or can be passed to a second separation unit 40 to generate a first recycle stream 42 and a second recycle stream 44. The first recycle stream 42 comprises olefins in the C4 to C14 range and is passed to the oligomerization reactor 20. The second recycle stream 44 has a reduced olefin content. The process can further include passing the second recycle stream 44 to a dehydrogenation reactor 50 to generate a dehydrogenation stream 52 having olefins. The dehydrogenation stream 52 can be passed to the oligomerization reactor for increasing the molecular weight of the olefins.

In one embodiment, the dehydrogenation stream 52 is passed to a selective hydrogenation reactor 60 to generate a dehydrogenation stream 62 having reduced diolefin and alkyne content. The subsequent stream 62 can be passed to the oligomerization reactor 20. The choice of oligomerization catalyst is preferably one that is relatively sulfur tolerant to enable the process to run with a feedstock having some sulfur in the feedstock.

The dehydrogenation stream 52, or the dehydrogenation stream with reduced diolefin and acetylene content 62 can be passed to a third separation unit 70 to generate a light stream 74 comprising C4 to C8 olefins, and a heavy stream 72 comprising C9 to C14 olefins. The heavy stream 72 is passed to a dimerization reactor 80 to generate a dimerization product stream 82. The dimerization product stream 82 can be passed to an alkylation reactor 90, where an aromatic stream 88 is passed to the reactor 90 to form an alkylaromatic process stream 92. The light stream 74 is passed to the oligomerization reactor 20.

In one embodiment the product stream 32 can be passed to the alkylation reactor 90. An aromatic stream 88 is passed to the alkylation reactor, where the reactor 90 is operated under alkylation conditions to generate an alkylaromatic process stream 92 comprising alkylaromatic compounds having alkyl group chains in the C15 to C36 range. The aromatic compounds can be benzene or toluene or mixtures thereof. The product stream 92 can comprise alkylbenzenes, or alkyltoluenes. The alkylaromatic process stream 92 can be passed to a separation unit to form an alkylaromatic product stream, and an aromatic process stream. The aromatic process stream can be recycled to the alkylation reactor 90.

One embodiment of the present invention is the integration of this invention into processes that generate hydrocarbons in the C4 to C6 range from a variety of sources. The process, as shown in Figure 2, includes extracting and processing hydrocarbons generated from oxygenated compounds, such as from a Fischer-Tropsch liquid. A feedstock 8 comprising olefins in the C4 to C6 range and oxygenated compounds is passed to an extraction unit 10. In the extraction unit 10, the feedstock 8 is contacted with a liquid extractant 6 to generate a deoxygenated feedstock 12. The deoxygenated feedstock 12 is passed to an oligomerization reactor 20 under chain growth conditions to provide for an oligomerization reactor process stream 22. The oligomerization reactor process stream 22 comprises heavy mono-olefins in the C15 to C36 range.

The liquid extractant stream 14 leaving the extraction unit 10 can be regenerated and recycled to the extraction unit 10. Extracted components can be passed to other processing units. The liquid extractant comprises at least one of alcohol and diol having 1 to 3 carbon atoms per molecule and a minor amount of water and is contacted with the olefin containing feedstock under extraction conditions. In one embodiment, the liquid extractant can comprise methanol and preferably contains less than 25 mass-percent water.

The oligomerization stream 22 is passed to a separation unit 30 to generate a heavy olefin product stream 32 and a second stream 34 having a reduced heavy olefin content. The heavy olefin product stream 32 is passed to an alkylation reactor 90 with a stoichiometric excess amount of an aromatic compound to alkylate the aromatic compound under alkylation conditions to generate an alkylation effluent stream 92 comprising alkylaromatics and benzene. The alkylation effluent stream 92 is passed to a separation unit 100 to generate an alkylaromatic product stream 102 and an aromatic stream 104. At least a portion of the aromatic stream 104 can be passed to the alkylation reactor 90.

The second stream 34 can be passed to a dehydrogenation reactor 50 to generate a third stream 52 comprising olefins. The third stream 52 is passed to the oligomerization reactor 20, wherein the third stream comprises between 1 and 50 mass-percent of C5 and C6 mono-olefins. In one embodiment, the third stream is passed to a selective hydrogenation unit 60 to reduce the amount of diolefins and acetylenes to generate an olefin stream 62 having a reduced diolefin content.

The aromatic compounds for alkylation can include benzene and toluene, with benzene preferred. For the surfactants used in enhanced oil recovery, the aromatic compounds can include other aromatic compounds such as monoethyl benzene, or other aromatic compounds with mono-substitution of small alkyl groups. This process can be incorporated into existing processes to utilize materials that might be unacceptable for detergent grade linear alkylbenzenes.

## Claims

1. A process for producing heavy olefins, comprising:
passing a hydrocarbon feedstock, comprising C4 to C6 olefins to an oligomerization reactor to generate a first stream comprising heavy olefins in the C15 to C36 range; and
passing the first stream to a first separation unit to generate a product stream comprising C15 to C36 olefins, and a recycle stream having a reduced heavy olefin content; the process further comprising:
passing a portion of the product stream to an alkylation reactor; and
passing an aromatic stream, comprising aromatic compounds, to the alkylation reactor to alkylate the aromatic compounds with the olefins under alkylation conditions to generate an alkylaromatic stream comprising alkylaromatic compounds having carbon chains in the C15 to C36 range on the aromatic groups.

2. The process of claim 1 wherein the hydrocarbon feedstock is a light cracked naphtha.

3. The process of claim 1 wherein the product stream comprises C18 to C28 olefins.

4. The process of claim 1 further comprising:
passing the recycle stream to a second separation unit to generate a first recycle stream comprising olefins in the C4 to C14 range and a second recycle stream having reduced olefin content; and
passing the first recycle stream to the oligomerization reactor.

5. The process of claim 4 further comprising:
passing the second recycle stream to a dehydrogenation unit to generate a dehydrogenation stream comprising C4 to C6 olefins.

6. The process of claim 5 further comprising:
passing the dehydrogenation stream to a selective hydrogenation reactor to generate a dehydrogenation stream with reduced alkyne and diolefin content; and
passing the dehydrogenation stream with reduced alkyne and diolefin content to the oligomerization reactor.

7. The process of claim 5 further comprising:
passing the dehydrogenation stream to a third separation unit to generate a light stream comprising C4 to C8 olefins and a heavy stream comprising C9 to C 14 olefins;
passing the heavy stream to a dimerization reactor to generate a dimerization reactor product stream comprising C18 to C28 olefins; and
passing the light stream to the oligomerization reactor.

8. The process of claim 1 further comprising separating the aromatic compounds from at least a portion of the alkylaromatic stream to generate an alkylaromatic product stream, and aromatics stream.

9. The process of claim 1 wherein the aromatic compounds comprise benzene and toluene.

## Patentansprüche

1. Prozess zur Herstellung schwerer Olefine, umfassend:
Einleiten eines Kohlenwasserstoffausgangsmaterials, das C4-C6-Olefine umfasst, in einen Oligomerisierungsreaktor, um einen ersten Strom zu erzeugen, der schwere Olefine im Bereich von C15 bis C36 umfasst; und
Einleiten des ersten Stroms in eine erste Trenneinheit, um einen Produktstrom, der C15-C36-Olefine umfasst, und einen Rücklaufstrom mit einem reduzierten Gehalt an schweren Olefinen zu erzeugen;
wobei der Prozess ferner umfasst:
Einleiten eines Teils des Produktstroms in einen Alkylierungsreaktor; und
Einleiten eines aromatischen Stroms, der aromatische Verbindungen umfasst, in den Alkylierungsreaktor, um die aromatischen Verbindungen mit den Olefinen unter Alkylierungsbedingungen zu alkylieren, um einen alkylaromatischen Strom zu erzeugen, der alkylaromatische Verbindungen mit Kohlenstoffketten im Bereich von C15 bis C36 auf den aromatischen Gruppen umfasst.

2. Prozess nach Anspruch 1, wobei Kohlenwasserstoffausgangsmaterial ein leicht gekracktes Naphtha ist.

3. Prozess nach Anspruch 1, wobei der Produktstrom C18-C28-Olefine umfasst.

4. Prozess nach Anspruch 1, ferner umfassend:
Einleiten des Rücklaufstroms in eine zweite Trenneinheit, um einen ersten Rücklaufstrom, der Olefine im Bereich von C4 bis C14 umfasst, und einen zweiten Rücklaufstrom mit reduziertem Olefingehalt zu erzeugen; und
Einleiten des ersten Rücklaufstroms in den Oligomerisierungsreaktor.

5. Prozess nach Anspruch 4, ferner umfassend:
Einleiten des zweiten Rücklaufstroms in eine Dehydrierungseinheit, um einen Dehydrierungsstrom zu erzeugen, der C4-C6-Olefine umfasst.

6. Prozess nach Anspruch 5, ferner umfassend:
Einleiten des Dehydrierungsstroms in einen selektiven Hydrierungsreaktor, um einen Dehydrierungsstrom mit reduziertem Alkyn- und Diolefingehalt zu erzeugen; und
Einleiten des Dehydrierungsstroms mit reduziertem Alkyn- und Diolefingehalt in den Oligomerisierungsreaktor.

7. Prozess nach Anspruch 5, ferner umfassend:
Einleiten des Dehydrierungsstroms in eine dritte Trenneinheit, um einen leichten Strom mit C4-C8-Olefinen und einen schweren Strom mit C9-C14-Olefinen zu erzeugen;
Einleiten des schweren Stroms in einen Dimerisierungsreaktor, um einen Dimerisierungsreaktor-Produktstrom zu erzeugen, der C18-C28-Olefine umfasst; und
Einleiten des leichten Stroms in den Oligomerisierungsreaktor.

8. Prozess nach Anspruch 1, ferner umfassend ein Trennen der aromatischen Verbindungen von mindestens einem Teil des alkylaromatischen Stroms, um einen alkylaromatischen Produktstrom und einen aromatischen Strom zu erzeugen.

9. Prozess nach Anspruch 1, wobei die aromatischen Verbindungen Benzol und Toluol umfassen.

## Revendications

1. Procédé pour la production d'oléfines lourdes, comprenant :
l'envoi d'une charge de départ d'hydrocarbures, comprenant des oléfines en C4 à C6, vers un réacteur d'oligomérisation pour produire un premier flux comprenant des oléfines lourdes dans la plage de C15 à C36 ; et
l'envoi du premier flux vers une première unité de séparation pour produire un flux de produit comprenant des oléfines en C15 à C36 et un flux de recyclage ayant une teneur réduite en oléfines lourdes ;
le procédé comprenant en outre :
l'envoi d'une partie du flux de produit vers un réacteur d'alkylation ; et
l'envoi d'un flux de composés aromatiques, comprenant des composés aromatiques, vers le réacteur d'alkylation pour alkyler les composés aromatiques avec les oléfines dans des conditions d'alkylation pour produire un flux de composés alkylaromatiques comprenant des composés alkylaromatiques ayant des chaînes carbonées dans la plage de C15 à C36 sur les groupes aromatiques.

2. Procédé selon la revendication 1 dans lequel la charge de départ d'hydrocarbures est un naphta craqué léger.

3. Procédé selon la revendication 1 dans lequel le flux de produit comprend des oléfines en C18 à C28.

4. Procédé selon la revendication 1 comprenant en outre :
l'envoi du flux de recyclage vers une deuxième unité de séparation pour produire un premier flux de recyclage comprenant des oléfines dans la plage de C4 à C14 et un second flux de recyclage ayant une teneur réduite en oléfines ; et
l'envoi du premier flux de recyclage vers le réacteur d'oligomérisation.

5. Procédé selon la revendication 4 comprenant en outre :
l'envoi du second flux de recyclage vers une unité de déshydrogénation pour produire un flux de déshydrogénation comprenant des oléfines en C4 à C6.

6. Procédé selon la revendication 5 comprenant en outre :
l'envoi du flux de déshydrogénation vers un réacteur d'hydrogénation sélective pour produire un flux de déshydrogénation à teneur réduite en alcynes et en dioléfines ; et
l'envoi du flux de déshydrogénation à teneur réduite en alcynes et en dioléfines vers le réacteur d'oligomérisation.

7. Procédé selon la revendication 5 comprenant en outre :
l'envoi du flux de déshydrogénation vers une troisième unité de séparation pour produire un flux de composés légers comprenant des oléfines en C4 à C8 et un flux de composés lourds comprenant des oléfines en C9 à C14 ;
l'envoi du flux de composés lourds vers un réacteur de dimérisation pour produire un flux de produit de réacteur de dimérisation comprenant des oléfines en C18 à C28 ; et
l'envoi du flux de composés légers vers le réacteur d'oligomérisation.

8. Procédé selon la revendication 1 comprenant en outre la séparation des composés aromatiques d'au moins une partie du flux de composés alkylaromatiques pour produire un flux de produit alkylaromatique et un flux de composés aromatiques.

9. Procédé selon la revendication 1 dans lequel les composés aromatiques comprennent du benzène et du toluène.
